# EUROPEAN PATENT APPLICATION

(11) **EP 2 216 000 A1**
(43) Date of publication of application: **11.08.2010**
(21) Application number: 08851907.9
(22) Date of filing: 18.08.2008
(51) Int. Cl.: A61F 13/15, A61F 13/49, A61F 13/494, A61F 13/511

(54) **ABSORBENT ARTICLE**

(30) Priority: 22.11.2007 JP 2007303731
(71) Applicant: Unicharm Corporation, Ehime 799-0111 (JP)
(72) Inventor: NAKAJIMA, Kaiyo, Kanonji-shi Kagawa 769-1602 (JP); MINATO, Hironao, Kanonji-shi Kagawa 769-1602 (JP)
(74) Representative: Stark, Gordon Drummond
(86) International application number: PCT/JP2008/064692
(87) International publication number: WO 2009/066493

(57) **Abstract**

The present invention aims to provide an absorbent article with a barrier sheet preventing body waste from coming in contact with a wearer's skin, on one hand, and to prevent an opening area of one or two through-holes formed in the barrier sheet from being reduced during use of the article, on the other hand.

An absorbent article including a barrier sheet 3 facing a wearer's skin and adapted to be spaced from a chassis in a crotch region and a pair of leak-barrier cuffs 4 attached to the barrier sheet 3 on its side facing a wearer's skin so as to extend in a front-to-rear direction Z. The barrier sheet 3 and the leak-barrier cuffs 4 are bonded together by means of joint zones 48. The joint zones 48 are located on respective free edges of the leak-barrier cuffs 4 which can be spaced from the chassis so as to overlap respective one of barrier sheet's elastic members 39.

## Description

### TECHNICAL FIELD

The present invention relates to absorbent articles and more particularly to absorbent articles such as disposable diapers, toilet-training pants or incontinent briefs.

### RELATED ART

Disposable diapers provided with skin-contact sheets serving to protect the wearers' skin from contact with body waste are known, for example, from the disclosure of JP2002-11044A (PATENT DOCUMENT 1). According to the disclosure of PATENT DOCUMENT 1, the diaper comprises a topsheet, a backsheet, an absorbent structure sandwiched between these topsheet and backsheet and a skin-contact sheet disposed on the side of the topsheet facing a wearer's skin wherein the skin-contact sheet is provided with at least one through-hole adapted to guide body waste to the side of the topsheet and elastic members attached under tension to the skin-contact sheet so as to surround the through-hole.
PATENT DOCUMENT 1: JP2002-11044A

### DISCLOSURE OF THE INVENTION

### PROBLEM TO BE SOLVED BY THE INVENTION

The foresaid diaper has a front-to-rear direction and a transverse direction and comprises front and rear waist regions and a crotch region extending between these front and waist regions. With this diaper of prior art put on the wearer's body, the crotch region bows in the transverse direction as the crotch region is squeezed between the wearer's groins and the skin-contact sheet is also squeezed inward as viewed in the transverse direction as the crotch region of the diaper bows in the transverse direction. As a result, an opening area of the through-hole is apt to be reduced.

The diaper disclosed in PATENT DOCUMENT 1 has a front-to-rear direction and a transverse direction and comprises front and rear waist regions and a crotch region extending between these front and waist regions. With this diaper of prior art put on the wearer' s body, the crotch region bows in the transverse direction as the crotch region is squeezed between the wearer's groins, and the skin-contact sheet is also squeezed inward as viewed in the transverse direction as the crotch region of the diaper bows in the transverse direction. As a result, an opening area of the through-hole is apt to be reduced.
The through-hole's contour describes a curve and the elastic member attached under tension so as to surround the through-hole also describes a corresponding curve. The curve initially describing such curve tends to be straighten as the elastic member contracts and to be displaced to reduce the opening area of the through-hole. When the opening area of the through-hole is reduced in this manner, an external genital of a wearer might come in contact with the peripheral edges of the through-hole and cause skin trouble derived from irritation due to such undesirable contact.

In view of the problem as has been described above, it is a principal object of the first aspect of the invention to provide a barrier sheet adapted to protect a wearer's skin from contact with body waste, on one hand, and to prevent the opening area of the through-hole formed on the barrier sheet from being reduced, on the other hand.

### MEASURE TO SOLVE THE PROBLEM

The invention relates to an improvement in an absorbent article comprising a chassis having a front-to-rear direction and a transverse direction, sides facing a wearer's skin and wearer's clothes, respectively, a front waist region, a rear waist region and a crotch region extending between the front and rear waist regions, and a liquid-absorbent structure disposed on the crotch region, a barrier sheet lying on the side of the chassis facing a wearer' s skin and provided in the crotch region so as to be spaced from the chassis and a pair of leak-barrier cuffs provided on the side of the barrier sheet facing a wearer's skin so as to be opposed to each other in the transverse direction and to extend in the front-to-rear direction, wherein the barrier sheet includes barrier sheet's elastic members extending in the front-to-rear direction and attached under tension to the barrier sheet and the liquid-absorbent structure is adapted to be bowed in a direction from the crotch region toward the front and rear waist regions and thereby to form a pocket between the barrier sheet and the liquid-absorbent structure.

This absorbent article being **characterized in that** the barrier sheet has a pair of lateral zones extending in the front-to-rear direction and opposed to each other in the transverse direction, a middle zone connecting the lateral zones to each other and through-holes defined by the pair of lateral zones and the middle zone so that body waste can pass the through-holes into the pocket, the leak-barrier cuffs comprise fixed edges bonded to the chassis, free edges not bonded to the chassis so as to be spaced from the chassis and cuff's elastic members attached under tension to the free edges and functioning to space the leak-barrier cuffs from the chassis, and the barrier sheet and the leak-barrier cuffs are bonded via joint zones located in the free edges and overlapping the barrier sheet's elastic members.

The invention relates also to an improvement in an absorbent article comprising a chassis having a front-to-rear direction and a transverse direction, sides facing a wearer's skin and wearer's clothes, respectively, a front waist region, a rear waist region and a crotch region extending between the front and rear waist regions, and a liquid-absorbent structure disposed on the crotch region, and a barrier sheet lying on the side of the chassis facing a wearer's skin and provided in the crotch region so as to be spaced from the chassis when the article is put on a wearer's body.

This absorbent article being **characterized in that** the barrier sheet has a pair of lateral zones extending in the front-to-rear direction and opposed to each other in the transverse direction, a middle zone connecting the lateral zones to each other, barrier sheet's elastic members attached under tension to the pair of lateral zones and extending in the front-to-rear direction, and front and rear ends extending in the transverse direction and opposed to each other in the front-to-rear direction wherein the front and rear ends are permanently bonded to the side of the chassis facing a wearer's skin, and the liquid-absorbent structure is adapted to be bowed in a direction from the crotch region toward the front and rear waist regions and thereby to form a pocket between the barrier sheet and the liquid-absorbent structure, the barrier sheet forms through-holes defined by the pair of lateral zones and the middle zone so that body waste can pass the through-holes into the pocket; the leak-barrier cuffs comprise fixed edges bonded to the chassis, free edges not bonded to the chassis being able to be spaced from the chassis and cuff's elastic members attached under tension to the free edges, the free edges is able to rise and collapse on the side of the chassis facing a wearer's skin thereby each of the free edges overlaps each of the lateral zones of the barrier sheet when these free edges are in collapsed state, and the barrier sheet is bonded to the leak-barrier cuffs in the free edges including the barrier sheet' s elastic members attached thereto, so that the lateral zones are displaced outward in the transverse direction as the leak-barrier cuffs raise themselves so that an opening area of the through-hole can be prevented from reduced in the transverse direction.

According to one preferred embodiment, the through-hole comprises, as viewed in the front-to-rear direction, a front through-hole lying on the front side with respect to the middle zone and a rear through-hole lying on the rear side with respect to the middle zone and the joint zones are provided in the lateral zones outside the front through-hole as viewed in the transverse direction.

According to another preferred embodiment, the front through-hole includes the transverse center line bisecting its dimension in the front-to-rear direction and the joint zones are provided aside from the center line toward the middle zone.

According to still another preferred embodiment, the barrier sheet includes the front and rear ends opposed to each other in the front-to-rear direction and respectively extending in the transverse direction, the front through-hole comprises the inner side edges lying in the lateral zones and the curved margin of closure extending along the middle zone so as to connect the inner side edges, the barrier sheet's elastic members include the curved segments extending along the curved margin of closure, and the joint zones are provided so as to overlap the curved segments.

According to yet another preferred embodiment, the leak-barrier cuffs are provided with auxiliary elastic members bonded under tension to the cuffs so as to extend in the front-to-rear direction of the free edges.

According to further another preferred embodiment, the auxiliary elastic members at least partially overlap the barrier sheet's elastic members.

### EFFECT OF THE INVENTION

According to the first aspect of the invention, the barrier sheet and the leak-barrier cuffs are bonded together via the joint zones provided so as to overlap the barrier sheet' s elastic members extending in the lateral zones of the barrier sheet. With such unique arrangement, the lateral zones of the barrier sheet are pulled outward as the leak-barrier cuffs are spaced upward from the chassis and therefore the opening area of the through-holes would not be reduced. Besides, the joint zones overlap the barrier sheet' s elastic members so as to exert a tensile force directly on the barrier sheet' s elastic members.

### BRIEF DESCRIPTION OF THE DRAWINGS

[FIG. 1] Fig. 1 is a perspective view showing the diaper as the first embodiment according to the first aspect of the invention.
[FIG. 2] Fig. 2 is a sectional view taken along a line II-II in Fig. 1.
[FIG. 3] Fig. 3 is a plan view showing the diaper of Fig. 1 as has been flatly developed.
[FIG. 4] Fig. 4 is a schematic diagram illustrating an important part as seen in Fig. 2.
[FIG. 5] Fig. 5 is a perspective view showing the parts illustrated by Fig. 4 as put on a wearer's body.
[FIG. 6] Fig. 6 is a sectional view taken along a line VI-VI in Fig. 4.
[FIG. 7] Fig. 7 is schematic diagram similar to Fig. 4, illustrating a second embodiment according to the first aspect of the invention.
[FIG. 8] Fig. 8 is a sectional view taken along a line VIII-VIII in Fig. 7.
[FIG. 9] Fig. 9 is a schematic diagram similar to Fig. 4, illustrating another embodiment according to the first aspect of the invention.
[FIG. 10] Fig. 10 is a schematic diagram similar to Fig. 4, illustrating still another embodiment according to the first aspect of the invention.

### IDENTIFICATION OF REFERENCE NUMERALS USED IN THE DRAWINGS

- 1: diaper
- 2: chassis
- 3: barrier sheet
- 4: leak-barrier cuffs
- 5: inner side
- 6: outer side
- 7: front waist region
- 8: rear waist region
- 9: crotch region
- 10: liquid-absorbent structure
- 27: front end
- 28: rear end
- 29: pocket
- 30: lateral zone
- 31: lateral zone
- 32: middle zone
- 33: front through-hole
- 34: rear through-hole
- 39: barrier sheet's elastic members
- 39b: curved segments
- 45: cuffs' elastic members
- 48: joint zones
- 50: first auxiliary elastic members
- 51: second auxiliary elastic members

### DESCRIPTION OF THE BEST MODE FOR WORKING OF THE INVENTION

The present invention will be exemplarily described hereinafter on the basis of the disposable diaper taken as typical embodiments of the absorbent article according to the present invention.

### <First embodiment according to the first aspect of the invention>

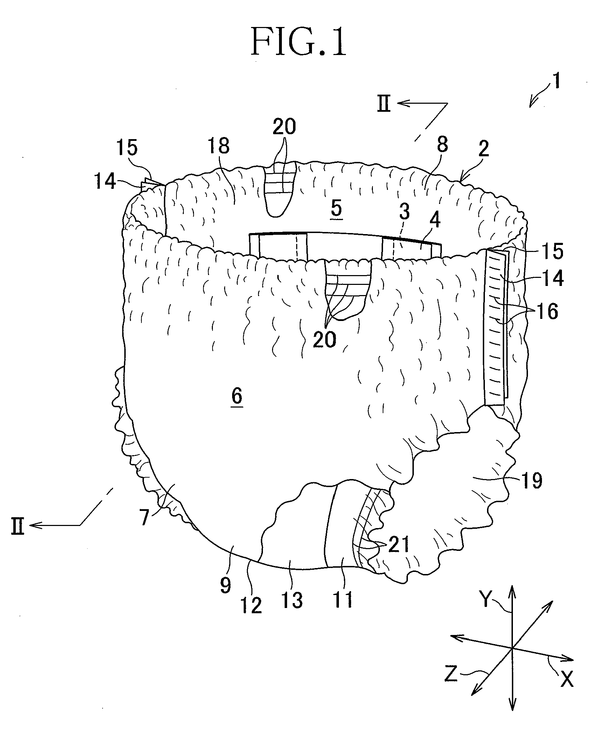

Figs. 1 through 6 illustrate a first embodiment according to the first aspect of the invention. Fig. 1 shows a diaper 1 as put on a wearer's body wherein the diaper 1 is partially cut off for convenience of illustration. As shown, the diaper 1 comprises a liquid-absorbent chassis 2, a barrier sheet 3 and leak-barrier cuffs 4. The chassis 2 is pants-shaped and defined by a transverse direction X, a vertical direction Y, a front-to-rear direction Z, an inner side 5 facing the wearer's skin, an outer side 6 facing the wearer's clothes, a front waist region 7, a rear waist region 8 and a crotch region 9 extending between said front and rear waist regions 7, 8. The chassis 2 comprises a topsheet 11, a backsheet 12 and a liquid-impervious leak-barrier sheet 13 sandwiched between these top- and backsheets 11, 12.

The front and rear waist regions 7, 8 respectively have pairs of side edges 14, 15 put flat and joined together at a plurality of joints 16 arranged intermittently along the respective side edges 14, 15 to form seams. In this way, the front and rear waist regions 7, 8 are joined together along the respective side edges 14, 15 so as to form a waist-opening 18 surrounded by the front and rear waist regions 7, 8 and a pair of leg-openings 19 surrounded by the respective rows of joints 16 and the crotch region 9. A plurality of waist elastic members 20 circumferentially extend along a peripheral edge of the waist-opening 18 and a plurality of leg elastic members 21 circumferentially extend along respective peripheral edges of the leg-openings 19. These elastic members 20, 21 are sandwiched between the topsheet 11 and the backsheet 12 and bonded under tension to at least one of these sheets 11, 12 by means of adhesive (not shown).

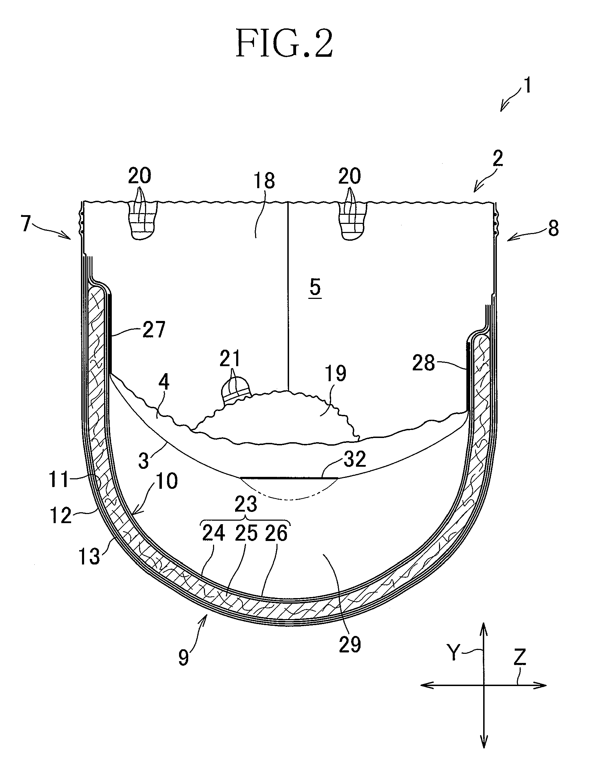

Fig. 2 is a sectional view taken along a line II-II in Fig. 1 and partially cut away for convenience of illustration. As will be seen in Fig. 2, the liquid-absorbent structure 10 is disposed on the inner side 5 of the chassis 2 facing a wearer's skin, the barrier sheet 3 is disposed on the side of the liquid-absorbent structure 10 facing the wearer' s skin, and the leak-barrier cuffs 4 is disposed on the side of the barrier sheet 3 facing the wearer's skin. While the liquid-absorbent structure 10 extends across the crotch region 9 and further into the front and rear waist regions 7, 8 in the front-to-rear direction Z in this embodiment, the liquid-absorbent structure 10 will effectively function so far as it extends at least across the crotch region 9. The liquid-absorbent structure 10 includes a liquid-absorbent panel 23 disposed on the inner surface of the topsheet 11.

The liquid-absorbent panel 23 comprises a liquid-absorbent core 25 wrapped with a liquid-absorbent and -spreadable sheet 24 such as tissue paper and an inner sheet 26 covering the liquid-absorbent and -spreadable sheet 24. The side of the liquid-absorbent panel 23 facing wearer's clothes is covered with the leak-barrier sheet 13 which is disposed on the inner surface of the backsheet 12 to prevent bodily fluids once absorbed by the core 25 from leaking out from the diaper 1. The leak-barrier sheet 13 may be bonded directly to the bottom surface of the liquid-absorbent panel 23.

The barrier sheet 3 is bonded in the vicinity of its front and rear ends 27, 28 to the inner sheet 26 by means of adhesion or sealing. The front end 27 and the rear end 28 are located in the front waist region 7 and the rear waist region 8, respectively. In a pants-shaped state as illustrated, the crotch region 9 is bowed and the barrier sheet 3 is spaced upward from the inner sheet 26 in the vertical direction Y under contraction of barrier sheet's elastic members which will be described later. The front and rear ends 27, 28 of the barrier sheet 3 are bonded to the inner sheet 26 so that the barrier sheet 3 as a whole takes a posture like a hammock being slung. As a result, a pocket 29 is defined between the barrier sheet 3 and the liquid-absorbent panel 23. The leak-barrier cuffs 4 are spaced upward from the barrier sheet 3 under contraction of cuffs' elastic members which will be described later and form a wall extending in the front-to-rear direction Z.

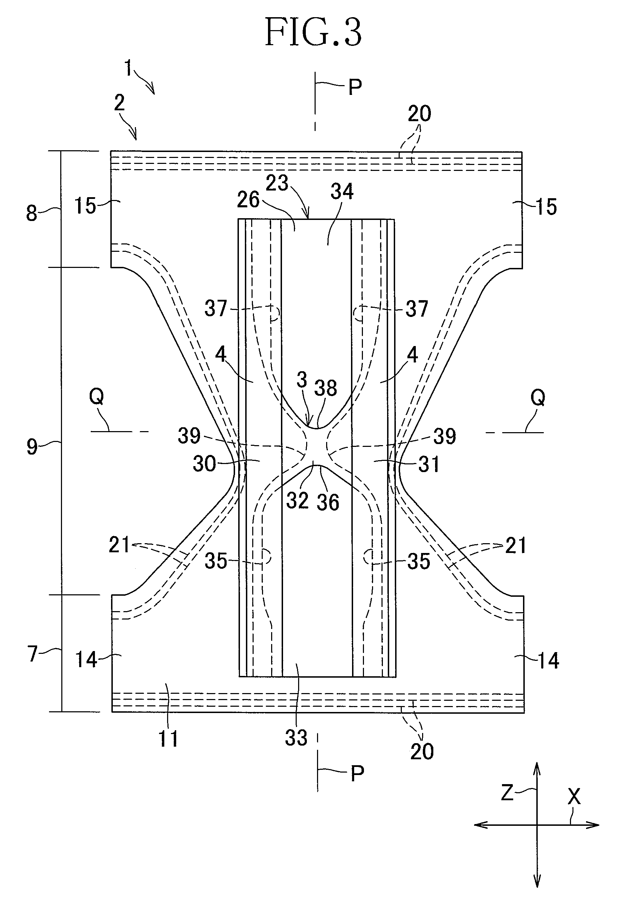

Fig. 3 is a plan view of the diaper 1 as flatly developed in the front-to-rear direction Z as well as in the transverse direction X after the front and rear waist regions 7 and 8 have been separated from each other along the respective rows of the joints 16 as seen in Fig. 1. In the state of the diaper 1 shown herein, the respective elastic members are under tension so as to keep the diaper 1 in the flattened state. As will be apparent from Fig. 3, the diaper 1 has a longitudinal center line P-P bisecting a dimension of the diaper 1 in the transverse direction X and a transverse center line Q-Q bisecting a dimension of the diaper 1 in the front-to-rear direction Y and is bilaterally-symmetric about the longitudinal center line P-P.

The chassis 2 has a concave shape curved inwardly and the liquid-absorbent panel 23 is rectangular and laminated on the topsheet 11 of the chassis 2. The barrier sheet 3 comprises a pair of lateral zones 30, 31 opposed to and spaced from each other in the transverse direction X and a middle zone 32 extending between these lateral zones 30, 31 wherein the middle zone 32 is located in the crotch region 9. The lateral zones 30, 31 cooperate with the middle zone 32 to form front and rear through-holes 33, 34. The front through-hole 33 has a substantially U-shape extending from the middle zone 32 into the front waist region 7 and the rear through-hole 34 has a substantially U-shape extending from the middle zone 32 into the rear waist region 8. The front through-hole 33 is defined by a pair of inner side edges 35 of the respective lateral zones 30, 31 and a curved margin of closure 36 connecting these inner side edges 35 to each other. The rear through-hole 34 is defined by a pair of inner side edges 37 of the respective lateral zones 30, 31 and a curved margin of closure 38 connecting these inner side edges 37 to each other. These curved margins of closure 36, 38 of the front and rear through-holes 33, 34 are defined by the middle zone 32 of the barrier sheet 3.

The leak-barrier cuffs 4 comprise a pair of sheets extending in the front-to-rear direction Z in symmetric relationship about the longitudinal center line P-P and formed, for example, by nonwoven fabric or plastic film which is preferably liquid-impervious. The leak-barrier cuffs 4 are formed so as to overlap respective skin-contacting surfaces of the lateral zones 30, 31 of the barrier sheet 3. Each of the leak-barrier cuffs 4 has a dimension in the transverse direction X substantially same as or larger than a dimension of the individual lateral zone 30, 31 of the barrier sheet 3 in the transverse direction X.

This diaper 1 may be put on a wearer's body with the wearer's external genital opposed to the front through-hole 33, the wearer's anus opposed to the rear trough-hole 34 and a zone of the wearer's skin defined between the external genital and the anus opposed to the middle zone 32. With the diaper 1 put on a wearer's body in this manner, the margin of closure 36 of the front through-hole 33 is preferably positioned aside forward from the transverse center Q-Q and the margin of closure 38 of the rear through-hole 34 is preferably positioned just on or in the vicinity of the transverse center line Q-Q. Thereupon, the liquid-absorbent panel 23 is bowed in the front-to-rear direction Z and the barrier sheet 3 lifted up into contact with a wearer's skin under contraction of the barrier sheet's elastic members 39 so that the pocket 29 (See Fig. 2) is formed between the barrier sheet 3 and the liquid-absorbent panel 23. In this way, body waste is reliably received by the pocket 29 through the front and rear through-holes 33, 34 and thus prevented from coming in direct contact with a wearer's skin.

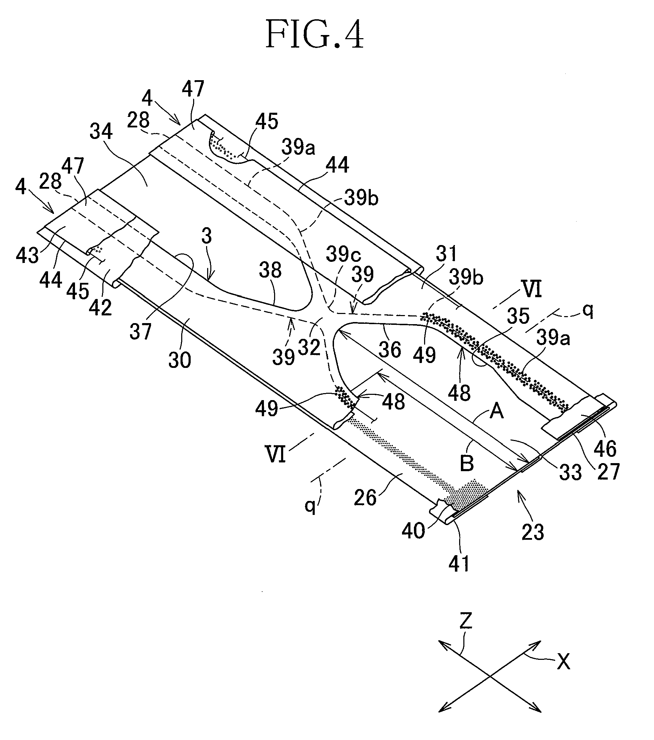

Fig. 4 is a diagram illustrating details of the liquid-absorbent panel 23, the barrier sheet 3 and the leak-barrier cuffs 4 but partially cutaway for convenience of illustration. The lateral zones 30, 31 of the barrier sheet 3 are folded back toward the liquid-absorbent panel 23 so as to define two-ply structures. Each of these two-ply lateral zones 30, 31 contains therein the barrier sheet' s elastic member 39 bonded thereto by means of adhesive (not shown). Specifically these barrier sheet's elastic members 39 are attached to the respective lateral zones 30, 31 under tension in the front-to-rear direction Z.

The front and rear through-holes 33, 34 have substantially U-shapes extending from the middle zone 32 into the front and rear waist regions 7, 8 defined by substantially rectilinear inner side edges 35, 37 and the curved margins of closure 36, 38. The barrier sheet's elastic members 39 extending along the front and rear through-holes 33, 34 respectively comprise rectilinear segments 39a extending in the vicinity of the inner side edges 35, 37, and curved segments 39a extending inward as viewed in the transverse direction X in the vicinity of the respective margins of closure 36, 38, as well as convex segments 39c extending along the middle zone 32 connecting the front and rear through-holes 33, 34 so as to be convex in the direction of the longitudinal center line P-P.

The two-ply lateral zones 30, 31 of the barrier sheet 3, at least the front and rear ends 27, 28 thereof are bonded to the inner sheet 26 of the liquid-absorbent panel 23 by means of adhesive 40. In the case of the lateral zones 30, 31 which are of so-called two ply structure as in this embodiment, the lateral zones 30, 31 can be bonded to the inner sheet 26 over respective entire ranges in the front-to-rear direction Z so that the barrier sheet 3 as a whole can move upward as the two-ply lateral zones 30, 31 are stretched.

Each of the leak-barrier cuffs 4 comprises a three-ply sheet having a Z-shaped cross section. More specifically, the leak-barrier cuff 4 comprises a first layer 41 sandwiched between the liquid-absorbent panel 23 and the chassis 2, a second layer 42 folded back from the first layer 41 onto the side of the barrier sheet 3 facing a wearer's skin so as to be placed upon the lateral zone 30 or 31 and a third layer 43 folded back from the second layer 42 onto the side of the second layer 42 facing the wearer's skin. The third layer 43 is folded back onto itself along a side edge 44 thereof so that a cuff's elastic member 45 may be attached thereto. The cuff's elastic member 45 is bonded between this two-ply side edge 44 under tension in the front-to-rear direction Z.

At front and rear ends 46, 47 of the leak-barrier cuff 4, the first layer 41 is bonded to the liquid-absorbent panel 23, the second layer 42 is bonded to the barrier sheet 3 and the third layer 43 is bonded to the second layer 42. Under contractile force of the cuff's elastic member 45 exerted on the leak-barrier cuff 4, the second layer 42 and the third layer 43 are unfolded and thereby spaced from the barrier sheet 3 so as to form a wall as seen in Fig. 2. A portion of the leak-barrier cuff 4 bonded to the topsheet 11 of the chassis 2 is referred to herein as a fixed edge and a portion comprising the second layer 42 and the third layer 43 of the leak-barrier cuff 4 adapted to be spaced from the barrier sheet 3, i.e. , not bonded to the barrier sheet 3 is referred to herein as a free edge.

The barrier sheet 3 and the leak-barrier cuffs 4 are bonded together along joint zones 48 each provided between the fixed edge and the free edge of the leak-barrier cuff 4 as viewed in the transverse direction X and partially overlapping the curved segment 39b of the associated barrier sheet's elastic member 39. More specifically, the joint zone 48 is formed by a belt of adhesive 49 extending from the front end 27 of the barrier sheet 3 in the front-to-rear direction Z by approximately 150 mm. In this manner, the belt of adhesive 49 continuously extends along the rectilinear segment 39a and the curved segment 39b. A dimension of the belt of adhesive 49 as measured in the transverse direction X is approximately 5 mm.

The joint zone 48 is preferably provided aside from a transverse center line q-q bisecting a dimension of the front through-hole 33 in the front-to-rear direction Z toward the middle zone 32. According to this embodiment, a dimension A as measured in the front-to-rear direction Z from the front end 27 to the margin of closure 36 of the front through-hole 33 is approximately 175 mm and a dimension B as measured in the front-to-rear direction Z from the front end 27 to the end of the joint zone 48 on the side of the middle zone 32 is approximately 150 mm. The dimension B is preferably about 88 mm or larger when the joint zone 48 is provided aside from the transverse center line q-q. The curved segment 39b of the barrier sheet's elastic member 39 describes a curve along the margin of closure 36 of the front through-hole 33. A curvature radius of this curved segment 39b is not constant and appropriately set so far as the curve is relatively gentle in the vicinity of the middle zone 32 and progressively becomes sharp along the inner side edge 35. Said joint zone 48 is preferably provided along the most sharply curved range.

While the dimension B may be approximately 88 mm or larger in the case of the dimensional relationship as has been described above, it is desired to set this dimension B to at least approximately 50 mm. This is for the reason that, if the dimension B is less than 50 mm, it would be difficult for the leak-barrier cuffs 4 to lift up the barrier sheet 3 effectively. Specifically, in each of the barrier cuffs 4, the first, second and third layers 41, 42, 43 are bonded together at the front end 46 and, in consequence, the leak-barrier cuff 4 will not be spaced from the barrier sheet 3 at the front end 46 at all and will be insufficiently spaced from the barrier sheet 3 in the vicinity of the front end 46. If the distance by which the leak-barrier cuff 4 can be spaced from the barrier sheet 3 is insufficient, a distance by which the barrier sheet 3 can be lifted up by the leak-barrier cuff 4 will be correspondingly insufficient. Consequently, it will not be ensured to prevent an opening area of the front through-hole 33 from being reduced.

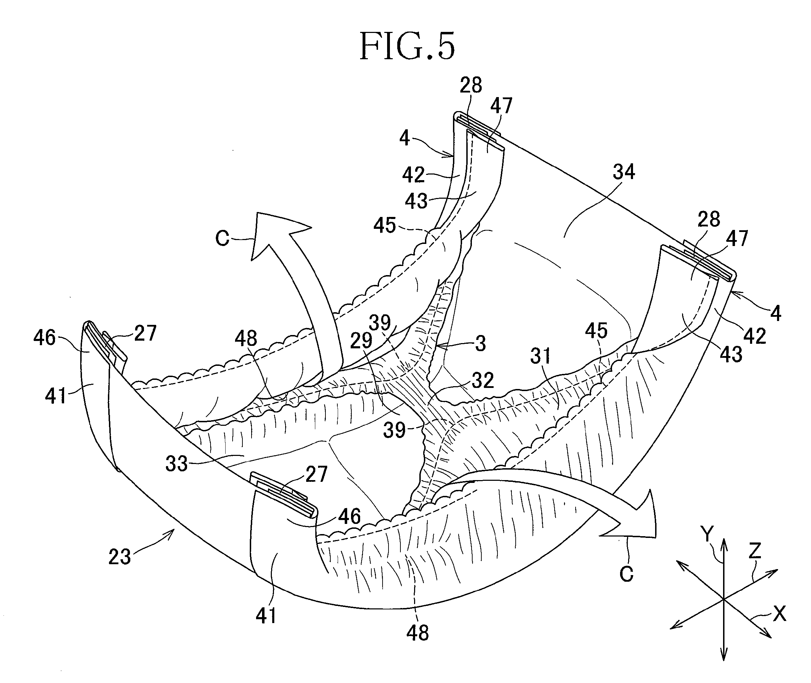

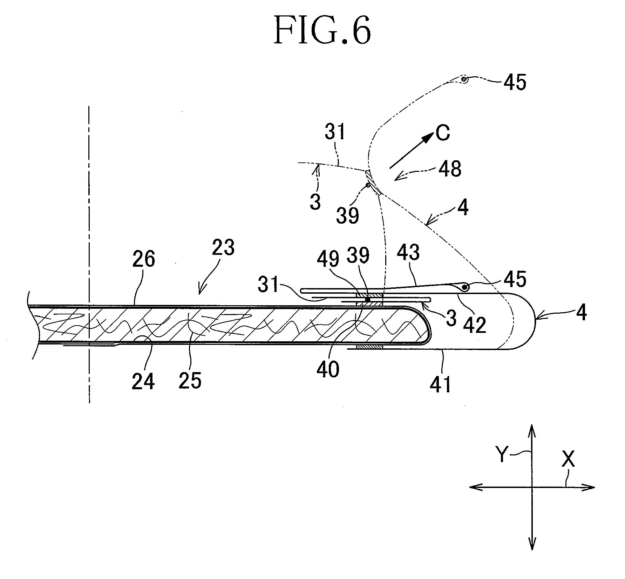

Fig. 5 is a perspective view showing the barrier sheet 3, the leak-barrier cuffs 4 and the liquid-absorbent panel 23 in the diaper put on the wearer's body and Fig. 6 is a sectional view taken along a line VI-VI in Fig. 4, wherein chain double-dashed lines indicate the diaper put on the wearer' s body. While Fig. 6 illustrates the lateral zone 31 of the barrier sheet 3, it will be appreciated that the lateral zone 30 is similar to the lateral zone 31.

Referring to Figs. 5 and 6, with the diaper put on a wearer's body, the barrier sheet's elastic member 39 as well as the cuff's elastic member 45 contracts. Upon contraction of the barrier sheet's elastic member 39, the liquid-absorbent panel 23 is bowed and the barrier sheet 3 is spaced upward from the liquid-absorbent panel 23. Upon contraction of the leak-barrier cuff's elastic member 45, the second layer 42 and the free edge of the third layer 43 are unfolded so as to be spaced from the liquid-absorbent panel 23, i.e., these layers raise themselves from their collapsed states. In response to the leak-barrier cuff 4 raising itself in this manner, the lateral zone 31 bonded to the second layer 42 in the joint zone 48 is lifted up so as to be spaced from the liquid-absorbent panel 23 outward as viewed in the transverse direction X and in a direction indicated by an arrow C. The lateral zone 31 lifted up in this manner serves to broaden the front through-hole 33 and thereby to prevent the opening area of the front through-hole 33 from being reduced even when the front through-hole 33 is squeezed between the wearer's legs, generating the force tending to reduce the opening area of the front through-hole 33.

The joint zone 48 formed at the location overlapping the barrier sheet's elastic member 39 allows a tensile force generated by the leak-barrier cuff 4 to be efficiently exerted on the barrier sheet 3. More specifically, the region of the barrier sheet 3 including the barrier sheet's elastic members 39 attached thereto is kept free from any sag because of contractile force of the barrier sheet's elastic member 39, but the region thereof including none of the barrier sheet' s elastic members 39 necessarily has a sag. Even when a tensile force is exerted on the slack region of the sheet, an amount by which the barrier sheet 3 as a whole can be lifted up will be insufficient to ensure that the tensile force generated by the cuff's elastic member 45 can be efficiently exerted on the barrier sheet 3.

The joint zone 48 formed so as to overlap the curved segment 39b of the barrier sheet's elastic member 39 further enhances the effect to prevent the opening area of the front through-hole 33 from being reduced. Normally the elastic member would tend to contract along the curved segment 39b back to its rectilinear state and thereby to bias the margin of closure 36 of the front through-hole 33 inward so as to reduce the opening area of the front through-hole 33. However, the joint zone 48 formed just on this curved segment 39b serves to restrain a movement of the barrier sheet's elastic member 39 tending to reduce the opening area of the front through-hole 33. The joint zone 48 is formed along the most sharply curved portion of the curved segment 39b in view of the fact that the biasing effect to reduce the opening area of the front through-hole 33 along this portion. It should be understood here that it is also possible to form the joint zone 48 along the rectilinear segment 39a rather than along the curved segment 39b depending on various factors such as a configuration of the barrier sheet's elastic member 39.

The barrier sheet 3 may be pulled from both sides to maintain its stretched state since the lateral zones 30, 31 of the barrier sheet 3 can be pulled outward in the transverse direction X via the joint zone 48. Maintaining the stretched state, the middle zone 32 of the barrier sheet 3 is prevented from sagging down as indicated by a dotted line in Fig. 2. So long as the barrier sheet 3 can be kept in its stretched state, the barrier sheet 3 can be reliably kept in contact with the wearer's skin following movement of the wearer's body. The barrier sheet 3 maintained in its stretched state allows also the liquid-absorbent panel 23 to be maintained in its curved state and thereby allows the body waste receiving pocket 29 to be reliably maintained.

While the joint zone 48 is formed by adhesive 49 applied in a belt-like shape according to this embodiment, the joint zone 48 may be formed by locally applying adhesive only along the curved segment 39b or the rectilinear segment 39a. While the joint zone 48 is provided only for the front through-hole 33, the joint zone 48 also may be formed for the rear through-hole 34. However, in view of the fact that the front through-hole 33 is opposed to the external genital and the rear through-hole 34 is opposed to the anus, the possibility that the opening area of the through-hole might be reduced is higher in the front through-hole 33 than in the rear through-hole 34. If the opening area of the front through-hole 33 is reduced, the front through-hole 33 may come in contact with the wearer's external genital and, in consequence, may cause skin trouble of the wearer.

While the barrier sheet's elastic member 39 including the curved segment 39b is used according to this embodiment, the invention is not limited to this and the elastic member 39 may not include the curved segment 39b or may rectilinearly extend in the front-to-rear direction. The elastic member 39 describing a V-shape convex toward the middle zone 32 may be also used. While the joint zone 48 is formed so as to overlap the barrier sheet's elastic member 39 according to this embodiment, it is not essential to form the joint zone 48 just on the barrier sheet's elastic member 39 and the joint zone 48 may be sufficiently near to the barrier sheet's elastic member 39 to ensure that the tensile force generated by the cuff's elastic member 45 is effectively exerted on the barrier sheet's elastic member 39. While there are provided the front and rear through-holes 33, 34 according to this embodiment, one of these front and rear through-holes 33, 34 may be eliminated or circumferentially closed in the front and rear waist regions 7, 8, respectively. In other words, these through-holes are not limited U-shaped through-holes but, for example, may be O-shaped, respectively.

As the adhesive 49 used to form the joint zone 48, rubber-based hot melt adhesive of well known art may be used. In addition to this, the joint zone 48 may be also formed using sealing technique such as sonic sealing or heat sealing technique. While the barrier sheet 3 and the leak-barrier cuff 4 respectively comprise single sheets according to this embodiment, it is possible to form them by adhesively joining a pair of sheets, respectively. In this case, the barrier sheet's elastic member 39 as well as the cuff's elastic member 45 may be sandwiched between a pair of sheets, respectively. Furthermore, the barrier sheet's elastic member 39 may be covered with the leak-barrier cuff 4 from above to ensure that a wearer's skin can be protected from coming in direct contact with the barrier sheet's elastic member 39 and thereby undesirable irritation to a wearer's skin can be alleviated.

### <Second embodiment according to the first aspect of the invention>

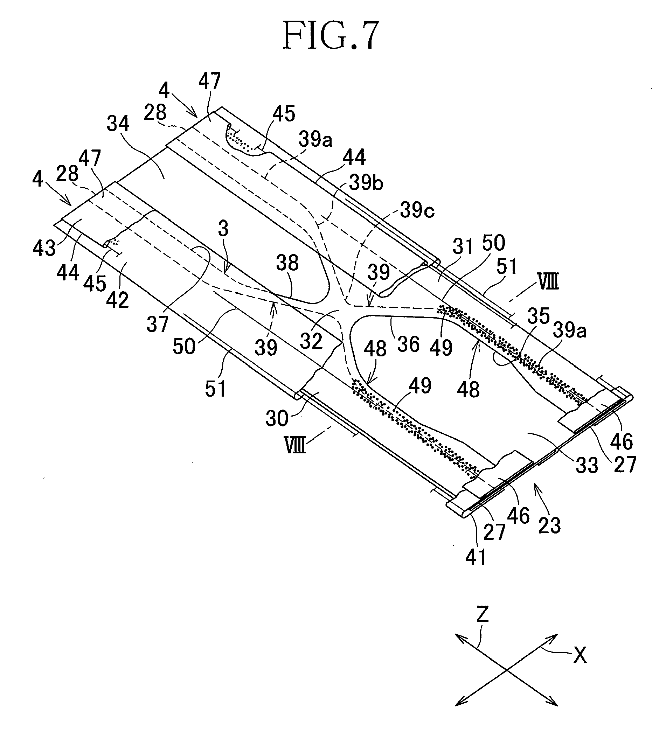

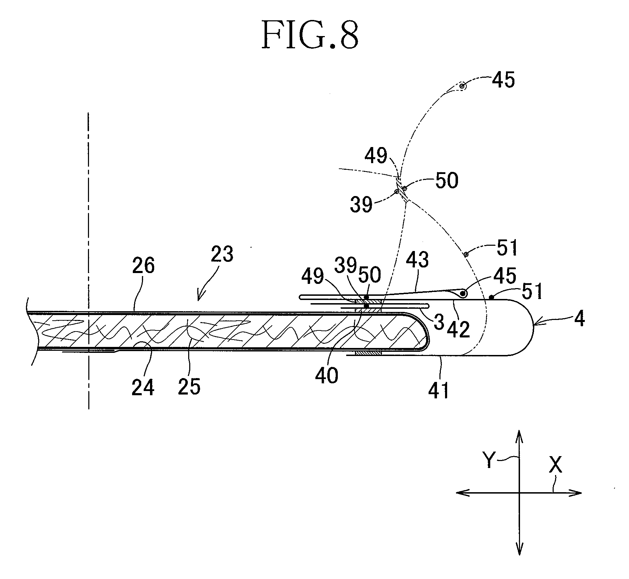

Figs. 7 and 8 illustrate a second embodiment according to the first aspect of the invention **characterized in that** the leak-barrier cuffs 4 are provided with auxiliary elastic members. The other features are similar to those in the first embodiment and will not be repetitively described. According to this embodiment, the auxiliary elastic member includes first and second auxiliary elastic members 50, 51 both extending in the front-to-rear direction Z of the leak-barrier cuffs 4, respectively.

As will be apparent from Fig. 7, the first and second auxiliary elastic members 50, 51 are attached to the respective second layers 42 of the leak-barrier cuffs 4 in a manner that the first auxiliary elastic member 50 is laid more inward than the second auxiliary elastic member 51 in the transverse direction X. The first and second auxiliary elastic members 50, 51 may be dimensioned in the front-to-rear direction Z so as to extend beyond the middle zone 32 to the joint zone 48. According to this embodiment, the first and second auxiliary elastic members 50, 51 are dimensioned to extend along the joint zone 48 to the front end 46. The first and second auxiliary elastic members 50, 51 are attached to the second layer 42 under tension in the front-to-rear direction Z. The first auxiliary elastic member 50 is attached to the second layer 42 so as to overlap the joint zone 48.

Fig. 8 is a sectional view taken along a line VIII-VIII in Fig. 7, wherein double-dashed lines indicate the state in which the barrier sheet's elastic members 39, the cuffs' elastic members 45, the first and second auxiliary elastic members 50, 51 contract. The first auxiliary elastic member 50 may be attached to the second layer 42 so as to overlap the joint zone 48 to ensure that the barrier sheet's elastic member 39, the adhesive 49 forming the joint zone 48 and the first auxiliary elastic member 50 are placed one upon another.

Contraction of the first auxiliary elastic member 50 causes the joint zone 48 to be pulled toward the middle zone 32 (See Fig. 7). In other words, the joint zone 48 is pulled rearward in the front-to-rear direction Z. The force pulling the joint zone 48 rearward functions to broaden the opening area of the front through-hole 33 in the vicinity of the margin of closure 36. According to this second embodiment, the joint zone 48 is pulled under contraction of the cuff's elastic member 45 and further pulled by the first auxiliary elastic member 50 so as to broaden the opening area of the front through-hole 33. In this way, it is further reliably ensured to prevent the opening area of the front through-hole 33 from being reduced.

The second auxiliary elastic member 51 is attached to the second layer 42 so as to extend substantially in parallel to and aside on the outer side of the first auxiliary elastic member 50 as viewed in the transverse direction X. Although this second auxiliary elastic member 51 is attached to the second layer 42 at a distance from the joint zone 48, the second auxiliary elastic member 51 is effective to pull the joint zone 48 rearward in the front-to-rear direction Z. In addition, the second auxiliary elastic member 51 is effective to prevent the leak-barrier cuff 4 particularly the second layer 42 thereof from slacking and thereby to ensure that the tensile force of the cuff's elastic member 45 is exerted on the joint zone 48.

While there are provided the first and second auxiliary elastic members 50, 51 according to this embodiment, it is not essential to incorporate both of these two elastic members. When one of these two elastic members is provided, it will be preferable to attach the first auxiliary elastic member 50 to the second layer 42 since the tensile force can be exerted thereby directly on the joint zone 48. Alternatively, it is also possible to provide three or more auxiliary elastic members. The first and second auxiliary elastic members 50, 51 may be used in the form of rubber strings, elasticized nonwoven fabric or the like.

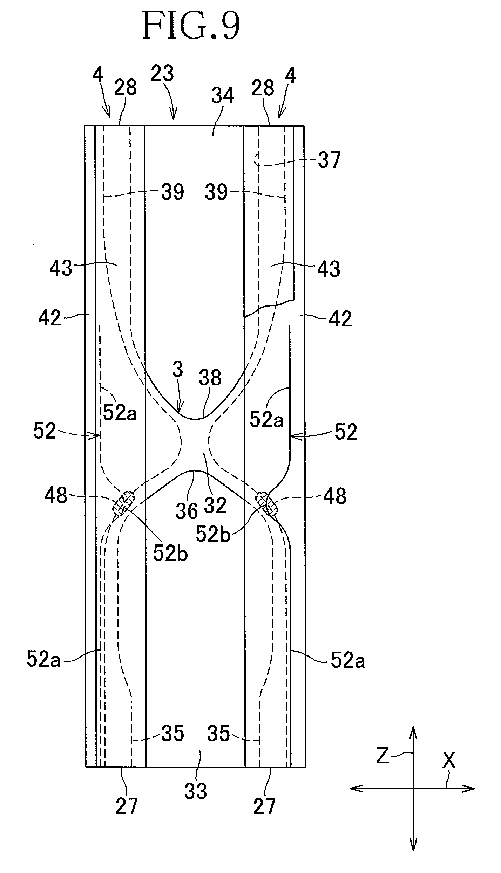

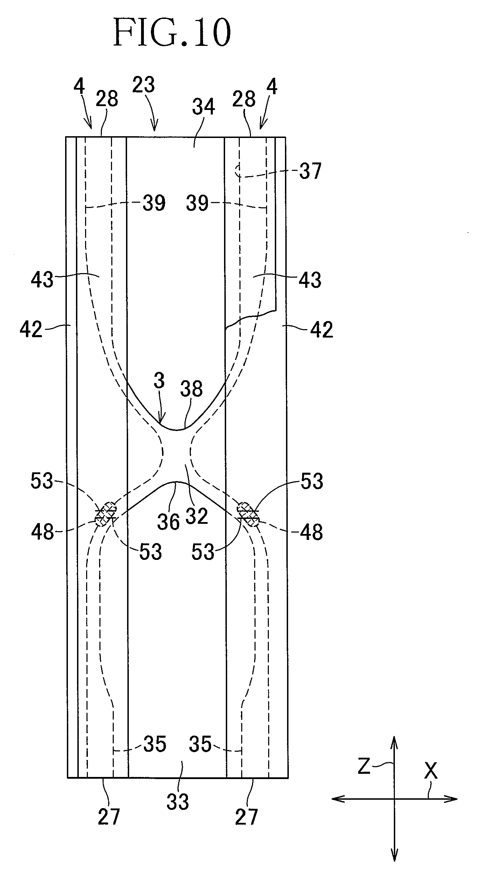

Figs. 9 and 10 illustrate other embodiments of the auxiliary elastic members. An auxiliary elastic member 52 used in the embodiment illustrated by Fig. 9 is attached under tension to the second layer 42 so as to extend across the joint zone 48 in the front-to-rear direction Z. More specifically, the auxiliary elastic member 52 comprises a rectilinear segment 52a and an oblique segment 52b wherein at least the oblique segment 52b overlaps the joint zone 48. Such manner of attachment ensures that not only a tensile force in the front-to-rear direction Z but also a tensile force in the transverse direction X is exerted on the joint zone 48. The tensile force in the transverse direction X functions to pull the inner edge 35 of the front through-hole 33 of the barrier sheet 3 outward as viewed in the transverse direction X and thereby to enhance the effect of preventing the opening area of the through-hole 33 from being reduced.

According to this embodiment, the joint zone 48 is not provided in a belt-like configuration but in a mass of adhesive locally applied at a distance from the front end 46. In accordance with this embodiment, both a particular angle and an orientation of the oblique segment 52b may be appropriately selected so far as a required tensile force in the transverse direction can be exerted on the joint zone 48.

The auxiliary elastic member 53 as illustrated in Fig. 10 is attached under tension to the leak-barrier cuff 4 so as to extend in the transverse direction X. Specifically a pair of the auxiliary elastic members 53 spaced from each other in the front-to-rear direction Z is attached to the leak-barrier cuff 4 and at least partially overlap the joint zone 48. Upon contraction of these auxiliary elastic members 53, the joint zone 48 is subjected to a tensile force pulling the zone 48 outward in the transverse direction X. Under such tensile force, the barrier sheet's elastic member 39 and the inner side edge 35 of the front through-hole 33 are also pulled outward and thereby the opening area of the front through-hole 33 could be prevented from being reduced.

According to the first aspect of the invention, materials conventionally used in the associated field of art can be used. For example, the barrier sheet 3 as well as the leak-barrier cuff 4 may be formed, e.g., by liquid-impervious and air-permeable nonwoven fabric, the topsheet 11 as well as the backsheet 12 may be formed, e.g., by air-permeable nonwoven fabric, the leak-barrier sheet 13 may be formed, e.g., by plastic film, and the liquid-absorbent core 25 included in the liquid-absorbent panel 23 may be formed, e.g., by a mixture of fluff pulp and super-absorbent polymer particles.

## Claims

1. An absorbent article comprising a chassis having a front-to-rear direction and a transverse direction, sides facing a wearer's skin and wearer's clothes, respectively, a front waist region, a rear waist region and a crotch region extending between said front and rear waist regions, and a liquid-absorbent structure disposed on said crotch region, a barrier sheet lying on said side of said chassis facing a wearer's skin and provided in said crotch region so as to be spaced from said chassis and a pair of leak-barrier cuffs provided on the side of said barrier sheet facing a wearer's skin so as to be opposed to each other in said transverse direction and to extend in said front-to-rear direction; wherein
said barrier sheet includes barrier sheet's elastic members extending in said front-to-rear direction and attached under tension to said barrier sheet; and
said liquid-absorbent structure is adapted to be bowed in a direction from said crotch region toward said front and rear waist regions and thereby to form a pocket between said barrier sheet and said liquid-absorbent structure,
said absorbent article being **characterized in that**:
said barrier sheet has a pair of lateral zones extending in said front-to-rear direction and opposed to each other in said transverse direction, a middle zone connecting said lateral zones to each other and through-holes defined by said pair of lateral zones and said middle zone so that body waste can pass said through-holes into said pocket;
said leak-barrier cuffs comprise fixed edges bonded to said chassis, free edges not bonded to said chassis so as to be spaced from said chassis and cuff's elastic members attached under tension to said free edges and functioning to space said leak-barrier cuffs from said chassis; and
said barrier sheet and said leak-barrier cuffs are bonded via joint zones located in said free edges and overlapping said barrier sheet's elastic members.

2. An absorbent article comprising a chassis having a front-to-rear direction and a transverse direction, sides facing a wearer's skin and wearer's clothes, respectively, a front waist region, a rear waist region and a crotch region extending between the front and rear waist regions, and a liquid-absorbent structure disposed on the crotch region, and a barrier sheet lying on the side of the chassis facing a wearer's skin and provided in the crotch region so as to be spaced from the chassis when the article is put on a wearer's body;
this absorbent article being **characterized in that** the barrier sheet has a pair of lateral zones extending in the front-to-rear direction and opposed to each other in the transverse direction, a middle zone connecting the lateral zones to each other, barrier sheet's elastic members attached under tension to the pair of lateral zones and extending in the front-to-rear direction, and front and rear ends extending in the transverse direction and opposed to each other in the front-to-rear direction wherein the front and rear ends are permanently bonded to the side of the chassis facing a wearer' s skin, and the liquid-absorbent structure is adapted to be bowed in a direction from the crotch region toward the front and rear waist regions and thereby to form a pocket between the barrier sheet and the liquid-absorbent structure, the barrier sheet forms through-holes defined by the pair of lateral zones and the middle zone so that body waste can pass the through-holes into the pocket; the leak-barrier cuffs comprise fixed edges bonded to the chassis, free edges not bonded to the chassis being able to be spaced from the chassis and cuff's elastic members attached under tension to the free edges, the free edges is able to rise and collapse on the side of the chassis facing a wearer' s skin thereby each of the free edges overlaps each of the lateral zones of the barrier sheet when these free edges are in collapsed state, and the barrier sheet is bonded to the leak-barrier cuffs in the free edges including the barrier sheet' s elastic members attached thereto, so that the lateral zones are displaced outward in the transverse direction as the leak-barrier cuffs raise themselves so that an opening area of the through-hole can be prevented from reduced in the transverse direction.

3. The article according to Claim 1 or 2, wherein said through-hole comprises, as viewed in said front-to-rear direction, a front through-hole lying on said front side with respect to said middle zone and a rear through-hole lying on said rear side with respect to said middle zone and said joint zones are provided in said lateral zones outside said front through-hole as viewed in said transverse direction.

4. The article according to Claim 3, wherein said front through-hole includes said transverse center line bisecting its dimension in said front-to-rear direction and said joint zones are provided aside from said center line toward said middle zone.

5. The article according to Claim 3 or 4, wherein said barrier sheet includes said front and rear ends opposed to each other in said front-to-rear direction and respectively extending in said transverse direction, said front through-hole comprises said inner side edges lying in said lateral zones and said curved margin of closure extending along said middle zone so as to connect said inner side edges, said barrier sheet's elastic members include said curved segments extending along said curved margin of closure, and said joint zones are provided so as to overlap said curved segments.

6. The article according to any one of Claims 1 through 5, wherein said leak-barrier cuffs are provided with auxiliary elastic members bonded under tension to said cuffs so as to extend in said front-to-rear direction of said free edges.

7. The article according to Claim 6, wherein said auxiliary elastic members at least partially overlap said barrier sheet' s elastic members.
